# EUROPEAN PATENT SPECIFICATION

(11) **EP 3 416 617 B1**
(45) Date of publication and mention of the grant of the patent: **18.12.2024**
(21) Application number: 17710482.5
(22) Date of filing: 17.02.2017
(51) Int. Cl.: A61K 31/46, A61K 31/4535, A61K 36/535, A61K 9/00, A61K 9/08, A61P 27/10

(54) **COMBINATION OF AN ANTIALLERGIC AGENT WITH MUSCARINIC ANTAGONIST AND/OR DOPAMINERGIC AGONIST FOR USE IN PREVENTING/STOPPING OF AXIAL MYOPIA IN HUMAN**
KOMBINATION EINES ANTIALLERGISCHEN MITTELS MIT MUSCARIN-ANTAGONIST UND/ODER DOPAMINERGEN-AGONIST ZUR VERWENDUNG BEIM VORBEUGEN/AUFHALTEN VON AXIALMYOPIE IN MENSCHEN
COMBINAISON D'UN AGENT ANTIALLERGIQUE AVEC UN ANTAGONISTE MUSCARINIQUE ET/OU UN AGONISTE DOPAMINERGIQUE POUR UTILISATION DANS LA PRÉVENTION/L'ARRÊT DE LA MYOPIE AXIALE CHEZ UN HUMAIN

(30) Priority: 19.02.2016 IT UB20160876
(43) Date of publication of application: 26.12.2018
(73) Proprietor: D'Ambrosio, Enzo Maria, 74024 Manduria (IT)
(72) Inventor: D'Ambrosio, Enzo Maria, 74024 Manduria (IT)
(74) Representative: Valenza, Silvia
(86) International application number: PCT/EP2017/053619
(87) International publication number: WO 2017/140846

(56) References cited:
- WO-A1-2012/090170
- LUFT W A ET AL: "Variable effects of previously untested muscarinic receptor antagonists on experimental myopia", INVESTIGATIVE OPHTHALMOLOGY & VISUAL SCIENCE - IOVS, ASSOCIATION FOR RESEARCH IN VISION AND OPHTHALMOLOGY, US, vol. 44, no. 3, 1 March 2003 (2003-03-01), pages 1330 - 1338, XP002315956, ISSN: 0146-0404, DOI: 10.1167/IOVS.02-0796
- PREMA GANESAN ET AL: "Pharmaceutical intervention for myopia control", EXPERT REVIEW OF OPHTHALMOLOGY, vol. 5, no. 6, 9 December 2010 (2010-12-09), GB, pages 759 - 787, XP055308425, ISSN: 1746-9899, DOI: 10.1586/eop.10.67
- AUDREY CHIA ET AL: "Atropine for the Treatment of Childhood Myopia: Safety and Efficacy of 0.5%, 0.1%, and 0.01% Doses (Atropine for the Treatment of Myopia 2)", OPHTHALMOLOGY, J. B. LIPPINCOTT CO., PHILADELPHIA, PA, US, vol. 119, no. 2, 20 July 2011 (2011-07-20), pages 347 - 354, XP028395893, ISSN: 0161-6420, [retrieved on 20110725], DOI: 10.1016/J.OPHTHA.2011.07.031
- MARTÍN ANDREA P ET AL: "The effect of ketotifen on inflammatory markers in allergic conjunctivitis: an open, uncontrolled study", BMC OPHTHALMOLOGY, BIOMED CENTRAL, LONDON, GB, vol. 3, no. 1, 6 January 2003 (2003-01-06), pages 2, XP021016312, ISSN: 1471-2415, DOI: 10.1186/1471-2415-3-2
- DEL CUVILLO ET AL: "Allergic Conjunctivitis and H 1 Antihistamines", J INVESTIG ALLERGOL CLIN IMMUNOL, 1 January 2009 (2009-01-01), pages 11 - 18, XP055308704, Retrieved from the Internet <URL:http://www.jiaci.org/issues/vol19s1/3.pdf> [retrieved on 20161009]

## Description

### FIELD OF THE INVENTION

The present invention relates to the field of combination of pharmaceutical active ingredient or multifunctional active principles for use in preventing/stopping of myopia in human.

### STATE OF THE ART

Myopia could be defined as the need for an eye of a negative lens to focus an image on the retinal plane. A number of subgroup of myopia exists, the most diffuse is the axial myopia, due to an excessive elongation of the ocular bulb. In most of western child the growth of the eye proceeds constantly, and the eye physiologically maintains a little farsightedness, but in some, mainly between 6-14 years, the ocular elongation is faster and results in nearsightedness. The underlining biological process is still poorly understood, genetic and ambiental factors contribute to its development. It's known that myopia is three times more prevalent in Asia than in western countries, some studies relate the development of the condition to the number of hours of indoor study or, inversely, to the time spent outside. In experimental model, chicks or small mammalian, excessive eye growth could be elicited by lid suture or degradation of the image using translucent lenses (form deprivation myopia or FDM) or, interestingly by the apposition of negative lenses (lens induced myopia or LIM). Are also available breeds of mice with a spontaneous abnormal eye growth.

Many do not consider myopia as a disease, because in most of cases is acceptably corrected by the means of glasses or contact lenses, but it could be intended as a disfunction of the eye growth. Moreover contact lens wearers are subject to allergic or foreign body reactions or more serious corneal infection that can cause loss of vision. Furthermore, a myopic eye is more subject to retinal degeneration and retinal tears that can lead to retinal detachment, glaucoma, myopic foveoschisis and macular hole, all causes of blindness. So effective therapy for preventing myopia is needed not only for esthetic finalities but also to prevent blinding disease in adult age.

Since the studies of R. Bedrossian (Ophthalmology, May 1979, Vol 86, pp. 713-717), a growing body of evidence shows that the blocking of M1 muscarinic receptor in the eye halt the axial elongation of the eye both in experimental models (Form deprivation myopia or lens induced myopia) and in human.

In the last 3 decades a number of studies explored the risks and benefits of Atropine eyedrops at various concentration or in association with other devices, like contact lenses or multifocal glasses, in order to find an effective formulation for Myopia prevention. These are analyzed in a Cochrane meta analysis (Walline JJ et al,Interventions to slow progression of myopia in children. Cochrane Database of Systematic Reviews 2011, Issue 12. Art. No.: CD004916). The better results were obtained from the ATOM (Atropine for Treatment Of Myopia) I and II studies. In the first study, published in 2006, (Chua W.H.. et al., Atropine for the Treatment of Childhood Myopia, Ophthalmology 2006;113:2285-2291) the authors demonstrated, on a placebo controlled large cohort of Chinese children, that the bedtime instillation of one drop of 1% Atropine collyrium halted the development of axial myopia in 90% of subjects, by stopping the elongation of the bulb. Since the main factor obstaculating the widespread use of the drug is the vision blurring due to mydriasis, also largely complained in ATOM I study, the same group tried in the second study (Chia A. et al, Atropine for the Treatment of Childhood Myopia: Safety and Efficacy of 0.5%, 0.1 %, and 0.01% Doses (Atropine for the Treatment of Myopia 2), Ophthalmology 2012;119:347-354) to reduce the dose to 0,1%, 0,05% and 0,01% demonstrating a dose dependent effect of atropine that is still acceptable at the lower concentration. By the way a small, around 5%, but still unacceptable number of subject, in a pediatric population, developed ocular redness and itching. This effect was proven triggered by an allergic response of the conjunctiva (Yoshikawa K., Kalahari S. Contact allergy to atropine and other mydriatic agents; CONTACT DERMATITIS 12(1):56 - 57, april 2006). The clinical uses of atropine in myopia control were also summarized in a review article (Ganesan P. et al., Pharmaceutical intervention for myopia control, Expert Rev. Ophthalmol., 2010; 5(6):759-787).

The stopping of eye elongation is not mediated by toxic effect on the retina, as postulated in some of the initial in vitro studies, further in vitro analysis and mfERG on human subject did not displayed any alteration on retinal function. (Chia A. et al. Full-field electroretinogram findings in children in the atropine treatment for myopia (ATOM2) study; Documenta Ophthalmologica 126(3) · January 2013).

Other muscarinic antagonists were tried, both on experimental model and human, in the attempt to reduce the side effects, in particular mydriasis. Principally pirenzepine was tried (EP 0478694 B1), but the effect correlated anyway to the degree of mydriasis and eye surface sensitization was more than with atropine, thus this way was abandoned. In another study (Luft W.A. et al., Variable effects of previously untested muscarinic receptor antagonists on experimental myopia, IOVS, 44(3):1330-1338), intravitreal injection of benztropine was shown to elicit a partial rescue in experimentally induced axial myopia. Its administration, however, was accompanied by signs of toxicity and inflammation.

Further studies, mainly in the last decade of the past century, explored more deeply the role of dopamine (DA) in the process of ocular axial elongation. In experimental models D1 agonists mainly enhanced the ocular elongation while D2 agonists and D1 antagonists block the process. Thus myopia could be explained as an imbalance between the two receptors activity. Results in human are not available and the development of eyedrops based on this pharmaceutical class is difficult because of poor solubility of the active principle at physiological pH. Human studies of Dopaminergic drug in human cannot be found, only experimental data is available, (Feldkaemper M. et al, An updated view on the role of dopamine in myopia, Experimental Eye Research 114 (2013) 106-119) and also dopamine reuptake inhibitors are active against experimental models of myopia induction. Dopamine agonists are nowadays used in Ophthalmology to restore a physiologic intraocular pressure (IOP) in severely compromised eyes, they have little effect on healthy eyes, and showed no risks in chronic use.

It is therefore apparent that one of the major issues against the use of muscarinic antagonists or dopamine agonist eyedrops for the controlling of eye growth and prevention of myopia is the unacceptable rate of iatrogenic conjunctivitis or dermatitis. Aim of the present invention is to provide an ophthalmic treatment for preventing/stopping myopia in children with relief of the observed side effects with muscarinic antagonists or dopamine agonist eyedrops.

### DEFINITION AND ABBREVIATIONS

AM: Axial myopia
FDM: Form deprivation myopia
LIM: Lens induced myopia
mfERG: multifocal Electroretinogram
DA: Dopamine
IOP: Intraocular pressure

### SUMMARY OF THE INVENTION

The present invention resolves the above problem by providing a combination of pharmaceutical active principles in a single formulation wherein an anticholinergic principle, selected from atropine or a salt thereof, is combined with an antihistaminic principle, selected-from ketotifen or fumarate salt thereof; or by providing a single pharmaceutical active principle having a combined activity as anticholinergic (Antimuscarinic) and antihistaminic, or as anticholinergic, antihistaminic and dopaminergic or monoamine reuptake inhibition, selected from benztropine or a salt thereof; for use in preventing/stopping axial myopia, wherein said combination or said single active principle are administered topically to the eye.

Surprisingly the above combination of principle/activity allows the prevention of eye axial elongation without giving rise to drawbacks, like mydriasis and allergic conjunctivitis or dermatitis, observed by the administration of atropine alone.

Further object of the present invention is therefore also an ophthalmic formulation, to be administrated topically to the eyes for use in preventing/stopping axial myopia comprising the combination of atropine or a salt thereof and ketotifen or the fumarate salt thereof; or benzotropine or benzotropine mesylate.

In a further embodiment, the present invention provides an ophthalmic formulation specially adapted for the topical administration to the eyes comprising atropine or a salt thereof and ketotifen or the fumarate salt thereof.

### DETAILED DESCRIPTION OF THE INVENTION

The present invention is defined by the claims. Any subject-matter falling outside the scope of the claims is provided for information only.

The combination of active principles or the single (antiparkinsonian) principle for use according to the invention are to be administered topically or locally to the eye. According to the invention the composition formulated for ophthalmic use may further comprise buffers, solubilizers (such as cyclodextrins, ionic or non ionic surfactants, phospholipidic micellae or similar, microsomes or others), gelificants (such as Hyaluronic acid, hydroxymethyl-cellulose, hydroxypropyl-cellulose, carboxymethylcellulose, Xantan gum, tamarind seed polysaccharide, povidone, carbopol and/or others) and preservatives (such as Benzalkonium chloride, benzoxonium chloride, cetylpyridinium, polyquad and/or others).

According to the invention the above composition can be a collyrium or a solution suitable for imbibition of a contact lens, or (with appropriate buffers) suitable for ocular iontophoresis or for the inclusion in intraocular, fornix or intrapunctal porous solid insert (biodegradable or not) like polylactate or similar polimers. Preferably, according to the invention the formulation can be sterile eyedrops with or without gelificant(s); a sterile solution suitable for contact lens impregnation; a sterile solution suitable for transscleral iontophoresis; a concentrated solution for impregnation of a solid porous device to be placed in the inferior conjunctival fornix for a sustained relase.

According to the invention the antihistaminic/antiallergic principle is ketotifen or the fumarate salt thereof.

According to the invention the anticholinergic principle is atropine base or salts thereof.

According to the invention the principle having a combined activity as anticholinergic and antihistaminic, or as anticholinergic, antihistaminic and dopaminergic (also as DA reuptake inhibitor) is Benztropine base or salts thereof as Benztropine methanesulfonate (mesylate).

A preferred combination for use according to the invention comprises atropine and Ketotifen fumarate.

A preferred antiparkinsonian principle for use as single active principle according to the invention is Benztropine mesylate.

Preferred buffers are Sodium phosphate monobasic and Sodium phosphate dibasic.

Preferred preservative is Benzalkonium chloride.

Another preferred ophthalmic formulation according to the invention comprises:

| | |
|---|---|
| - atropine sulphate | 0.01% - 1.0%, |
| - Ketotifen fumarate | 0.01%-0.1% |
| - Sodium phosphate monobasic | 0.05 M, |
| - Sodium phosphate dibasic | 0.05M, |
| - Benzalkonium chloride | 0.025% - 0.1%, |
| - purified water | as remainder |

wherein the percentages are based on the total weight of the composition.

Another preferred ophthalmic formulation according to the invention comprises:

| | |
|---|---|
| benztropine mesylate | 0.001% - 3.0%, |
| - Sodium phosphate monobasic | 0.05 M, |
| - Sodium phosphate dibasic | 0.05M, |
| - Benzalkonium chloride | 0.025% - 0.1 %, |
| - purified water | as remainder |

wherein the percentages are based on the total weight of the composition.

## Claims

1. A combination of pharmaceutical active principles or a single pharmaceutical active principle to be administered topically to the eyes for use in preventing/stopping axial myopia, wherein said combination comprises atropine or a salt thereof and Ketotifen or the fumarate salt thereof;
wherein said single active principle is Benztropine or a salt thereof.

2. The combination for use according to claim 1 comprising atropine sulphate and Ketotifen fumarate.

3. The single pharmaceutical active principle for use according to claim 1 which is selected in the group consisting of Benztropine or Benztropine mesylate.

4. An ophthalmic formulation to be administered topically to the eyes for use in preventing/stopping axial myopia comprising the combination of atropine or a salt thereof and Ketotifen or the fumarate salt thereof; or the single active principle Benztropine or Benztropine mesylate.

5. The ophthalmic formulation for use according to claim 4 in form of a collyrium or a solution suitable for imbibition of a contact lens, or suitable for ocular iontophoresis or for the inclusion in intraocular, fornix or intrapunctal porous solid insert consisting of polylactate or similar polymers.

6. The ophthalmic formulation for use according to claim 5 in form of sterile eyedrops with or without gelificant(s); a sterile solution suitable for contact lens impregnation; a sterile solution suitable for transscleral iontophoresis; a concentrated solution for impregnation of a solid porous device to be placed in the inferior conjunctival fornix for a sustained release.

7. Ophthalmic formulation for use according to any one of claims 4-6 comprising the combination of atropine sulphate and Ketotifen fumarate, or the single active principle benztropine or benztropine mesylate

8. The ophthalmic formulation, the combination or the single active principle for use according to any one of claims 2-4 further comprising buffers, a solubilizer, gelificants and/or preservatives.

9. The ophthalmic formulation, the combination or the single active principle for use according to claim 8 wherein buffers are Sodium phosphate monobasic and Sodium phosphate dibasic and/or preservative is Benzalkonium chloride.

10. The topical ophthalmic formulation for use according to claim 9 comprising
0.001-3.0% Benztropine methanesulfonate (mesylate);
0.05 M Sodium phosphate monobasic;
0.05 M Sodium phosphate dibasic;
0.025% to 0.1 % Benzalkonium chloride; and
purified water as remainder, wherein the percentages are based on the total weight of the composition.

11. The topical ophthalmic formulation for use according to claim 9 comprising
0,01-1,0% atropine sulphate
0.01-0.1% Ketotifen fumarate
0.05 M Sodium phosphate monobasic;
0.05 M Sodium phosphate dibasic;
0.025% to 0.1 % Benzalkonium chloride; and
purified water as remainder, wherein the percentages are based on the total weight of the composition.

12. An ophthalmic formulation specially adapted for the topical administration to the eyes comprising atropine or a salt thereof and Ketotifen or the fumarate salt thereof.

13. The ophthalmic formulation according to claim 12 which is as defined in any one of claims 5-9 or 11.

## Patentansprüche

1. Kombination aus pharmazeutischen Wirkstoffen oder ein einzelner pharmazeutischer Wirkstoff zur topischen Verabreichung an die Augen zur Verwendung beim Vorbeugen/Aufhalten von Axialmyopie, wobei die Kombination Atropin oder ein Salz davon und Ketotifen oder das Fumaratsalz davon umfasst;
wobei der einzelne Wirkstoff Benztropin oder ein Salz davon ist.

2. Kombination zur Verwendung nach Anspruch 1, die Atropinsulfat und Ketotifenfumarat umfasst.

3. Einzelner pharmazeutischer Wirkstoff zur Verwendung nach Anspruch 1, der aus der Gruppe ausgewählt ist, die aus Benztropin oder Benztropinmesylat besteht.

4. Ophthalmische Formulierung zur topischen Verabreichung an die Augen zur Verwendung beim Vorbeugen/Aufhalten von Axialmyopie, die die Kombination von Atropin oder einem Salz davon und Ketotifen oder dem Fumaratsalz davon oder den einzelnen Wirkstoff Benztropin oder Benztropinmesylat umfasst.

5. Ophthalmische Formulierung zur Verwendung nach Anspruch 4 in Form von Augenwasser oder einer Lösung, die zum Tränken einer Kontaktlinse geeignet ist, oder zur okulären Iontophorese oder für die Einbindung in ein intraokulares, Fornix- oder intrapunctales poröses festes Insert, bestehend aus Polylactat oder ähnlichen Polymeren, geeignet ist.

6. Ophthalmische Formulierung zur Verwendung nach Anspruch 5 in Form von sterilen Augentropfen mit oder ohne Geliermittel(n); einer sterilen Lösung, die für die Imprägnierung von Kontaktlinsen geeignet ist; einer sterilen Lösung, die für die transsklerale Iontophorese geeignet ist; einer konzentrierten Lösung zur Imprägnierung einer festen porösen Vorrichtung, die in die untere Fornix conjunctivae für eine verzögerte Freisetzung eingebracht wird.

7. Ophthalmische Formulierung zur Verwendung nach einem der Ansprüche 4-6, die die Kombination von Atropinsulfat und Ketotifenfumarat oder den einzelnen Wirkstoff Benztropin oder Benztropinmesylat umfasst.

8. Ophthalmische Formulierung, die Kombination oder der einzelne Wirkstoff zur Verwendung nach einem der Ansprüche 2-4, die/der ferner Puffer, einen Lösungsvermittler, Geliermittel und/oder Konservierungsmittel umfasst.

9. Ophthalmische Formulierung, die Kombination oder der einzelne Wirkstoff zur Verwendung nach Anspruch 8, wobei die Puffer monobasisches Natriumphosphat und dibasisches Natriumphosphat sind und/oder das Konservierungsmittel Benzalkoniumchlorid ist.

10. Topische ophthalmische Formulierung zur Verwendung nach Anspruch 9, umfassend
0,001-3,0 % Benztropinmethansulfonat (Mesylat);
0,05 M monobasisches Natriumphosphat;
0,05 M dibasisches Natriumphosphat;
0,025 % bis 0,1 % Benzalkoniumchlorid; und
gereinigtes Wasser als Rest, wobei sich die Prozentanteile auf das Gesamtgewicht der Zusammensetzung beziehen.

11. Topische ophthalmische Formulierung zur Verwendung nach Anspruch 9, umfassend
0,01-1,0 % Atropinsulfat
0,01-0,1 % Ketotifenfumarat
0,05 M monobasisches Natriumphosphat;
0,05 M dibasisches Natriumphosphat;
0,025 % bis 0,1 % Benzalkoniumchlorid; und
gereinigtes Wasser als Rest, wobei sich die Prozentanteile auf das Gesamtgewicht der Zusammensetzung beziehen.

12. Ophthalmische Formulierung, die insbesondere zur topischen Verabreichung an die Augen geeignet ist und Atropin oder ein Salz davon und Ketotifen oder das Fumaratsalz davon umfasst.

13. Ophthalmische Formulierung nach Anspruch 12, die wie in einem der Ansprüche 5-9 oder 11 definiert ist.

## Revendications

1. Combinaison de principes actifs pharmaceutiques ou principe actif pharmaceutique unique à administrer par voie topique aux yeux à utiliser dans la prévention/l'arrêt de la myopie axiale, dans laquelle ladite combinaison comprend de l'atropine ou un sel de celle-ci et du Kétotifène ou son sel de fumarate ;
dans laquelle ledit principe actif unique est la Benztropine ou un sel de celle-ci.

2. Combinaison à utiliser selon la revendication 1, comprenant du sulfate d'atropine et du fumarate de Kétotifène.

3. Principe actif pharmaceutique unique à utiliser selon la revendication 1 qui est choisi dans le groupe constitué par la Benztropine ou la Benztropine mésylate.

4. Formulation ophtalmique à administrer par voie topique aux yeux à utiliser dans la prévention/l'arrêt de la myopie axiale comprenant la combinaison d'atropine ou d'un sel de celle-ci et de Kétotifène ou de son sel de fumarate ; ou le principe actif unique Benztropine ou Benztropine mésylate.

5. Formulation ophtalmique à utiliser selon la revendication 4 sous la forme d'un collyre ou d'une solution appropriée pour l'imbibition d'une lentille de contact, ou appropriée pour l'iontophorèse oculaire ou pour l'inclusion dans un insert solide poreux intraoculaire, fornix ou intrapunctal constitué de polylactate ou de polymères similaires.

6. Formulation ophtalmique à utiliser selon la revendication 5 sous forme de gouttes oculaires stériles avec ou sans gélifiant(s) ; une solution stérile adaptée à l'imprégnation des lentilles de contact ; une solution stérile adaptée à l'iontophorèse transsclérale ; une solution concentrée pour l'imprégnation d'un dispositif poreux solide à placer dans le fornix conjonctival inférieur pour une libération prolongée.

7. Formulation ophtalmique à utiliser selon l'une quelconque des revendications 4 à 6, comprenant la combinaison de sulfate d'atropine et de fumarate de Kétotifène, ou le principe actif unique benztropine ou benztropine mésylate.

8. Formulation ophtalmique, la combinaison ou le principe actif unique à utiliser selon l'une quelconque des revendications 2 à 4, comprenant en outre des tampons, un solubilisant, des gélifiants et/ou des conservateurs.

9. Formulation ophtalmique, la combinaison ou le principe actif unique à utiliser selon la revendication 8, dans laquelle les tampons sont le phosphate de sodium monobasique et le phosphate de sodium dibasique et/ou le conservateur est le chlorure de Benzalkonium.

10. Formulation ophtalmique topique à utiliser selon la revendication 9 comprenant
0,001 à 3,0 % de méthanesulfonate de Benztropine (mésylate) ;
0,05 M de Phosphate de sodium monobasique ;
0,05 M de Phosphate de sodium dibasique ;
0,025 % à 0,1 % de chlorure de benzalkonium ; et
le reste étant constitué d'eau purifiée, dans laquelle les pourcentages sont basés sur le poids total de la composition.

11. Formulation ophtalmique topique à utiliser selon la revendication 9 comprenant
0,01 à 1,0 % de sulfate d'atropine
0,01 à 0,1 % de fumarate de Kétotifène
0,05 M de Phosphate de sodium monobasique ;
0,05 M de Phosphate de sodium dibasique ;
0,025 % à 0,1 % de chlorure de Benzalkonium ; et
le reste étant constitué d'eau purifiée, dans laquelle les pourcentages sont basés sur le poids total de la composition.

12. Formulation ophtalmique spécialement adaptée pour l'administration topique aux yeux comprenant de l'atropine ou un sel de celle-ci et du Kétotifène ou son sel de fumarate.

13. Formulation ophtalmique selon la revendication 12 qui est telle que définie dans l'une quelconque des revendications 5 à 9 ou 11.
